# FASCICULE DE BREVET EUROPEEN

(11) **EP 1 084 695 B1**
(45) Date de publication et mention de la délivrance du brevet: **05.11.2008**
(21) Numéro de dépôt: 00402374.3
(22) Date de dépôt: 28.08.2000
(51) Int. Cl.: A61Q 5/06, A61Q 1/10, A61K 8/894, A61K 8/73

(54) **Composition cosmétique comprenant au moins un copolymère silicone/acrylate et au moins un agent épaississant non-cellulosique**
Kosmetische Zusammensetzung enhaltend mindestens ein Silikon/Acrylat Copolymer und mindestens ein nichtzellulosiches Verdickungsmittel
Cosmetic composition containing at least a silicone/acrylate copolymer and at least a non-cellulosic thickening agent

(30) Priorité: 16.09.1999 FR 9911598
(43) Date de publication de la demande: 21.03.2001
(73) Titulaire: L'ORÉAL, 75008 Paris (FR)
(72) Inventeur: Dupuis, Christine, 75018 Paris (FR)
(74) Mandataire: Bourdeau, Françoise

(56) Documents cités:
- EP-A- 0 408 311
- WO-A-01/13884
- WO-A-99/04750

## Description

L'invention a pour objet une composition cosmétique, comprenant au moins un copolymère silicone/acrylate particulier et au moins un agent épaississant. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

On connaît des compositions épaissies, notamment des gels capillaires, qui sont généralement appliquées sur les cheveux avant de mettre en forme la coiffure. Pour mettre en forme et fixer la coiffure, on effectue ensuite un brushing ou une mise en plis. Ces compositions épaissies, notamment ces gels peuvent contenir des résines polymères.

Toutefois, elles présentent souvent l'inconvénient d'altérer les propriétés cosmétiques des cheveux. Ainsi, les cheveux peuvent devenir rêches, difficiles à démêler, perdre leur toucher et leur aspect_agréables ou encore manquer de corps. On recherche donc de telles compositions cosmétiques capillaires procurant de bonnes propriétés cosmétiques, notamment en terme de démêlage, de douceur et de toucher.

En outre, les compositions cosmétiques capillaires épaissies présentent également l'inconvénient majeur de donner lieu à une effet de poudrage. Au sens de la présente invention, on entend par « poudrage », l'aptitude du matériau obtenu par séchage de la composition capillaire à former une poudre après son application sur les cheveux. Bien entendu, la poudre tombe sur les épaules ou les vêtements de l'utilisateur, ou encore elle accroche au peigne ou à la brosse, et ceci est préjudiciable.

Il existe donc un besoin de trouver des compositions cosmétiques capillaires épaissies, notamment des gels, qui ne présentent pas l'ensemble des inconvénients indiqués ci-dessus, et qui en particulier fixent bien la coiffure, tout en procurant de bonnes propriétés cosmétiques, ceci sans effet de poudrage.

De manière surprenante et inattendue, la Demanderesse a découvert que lorsque l'on associe des copolymères silicone/acrylate avec certains agents épaississants, il est possible d'obtenir des compositions cosmétiques répondant aux exigences exprimées ci-dessus.

L'invention a pour objet une composition cosmétique capillaire comprenant, dans un milieu cosmétiquement acceptable, au moins un copolymère silicone/acrylate et au moins un agent épaississant non cellulosique, le copolymère silicone/acrylate étant obtenu par polymérisation radicalaire d'au moins un monomère éthyléniquement insaturé (a) en présence d'au moins un dérivé siliconé (b) comprenant des groupements oxyalkylénés, l'agent épaississant étant choisi parmi les gommes de xanthane, de scléroglucane, de gellane, de rhamsan, les alginates, la maltodextrine, l'amidon et ses dérivés, la gomme de karaya, la farine de caroube et gommes de guar.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition pour la fabrication d'une formulation cosmétique capillaire, destinée notamment au maintien et/ou à la mise en forme de la coiffure.

Les copolymères silicone/acrylate particulièrement visés par la présente invention sont ceux décrits dans la demande internationale WO99/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule (Iₐ) : 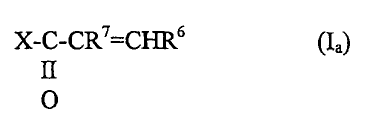 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH ⁴⁺, alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

En particulier, les monomères (a) de formule (la) sont l'acide acrylique et ses sels, esters ou amides. Les esters peuvent être dérivés d'alkyles linéaires en C₁ à C₄₀, d'alkyles ramifiés en C₃ à C₄₀ ou d'alcools carboxyliques en C₃ à C₄₀, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (la) peuvent également être choisis parmi les N,N-dialkylaminoalkyl acrylates et méthacrylates et N-dialkylaminoalkylacryl- et -méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en C₁ à C₄₀ ou ramifiées en C₃ à C₄₀.

Les monomères (a) de formule (la) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les bases oraganiques telles que les aminoalcools, comme les alcanolamines tels que la méthanolamine, le 2-amino-2-méthyl-l-propanol, la triéthanolamine, et les diamines, comme la lysine.

Les monomères (a) peuvent être aussi quaternisés lorsqu'ils possèdent un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning.

Parmi les monomères (b), on préfère ceux qui présentent la formule I ci-après : 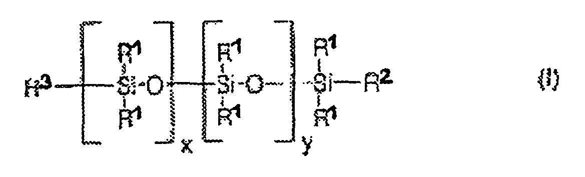 dans laquelle formule (I):
- R² est choisi parmi CH₃ ou le groupement 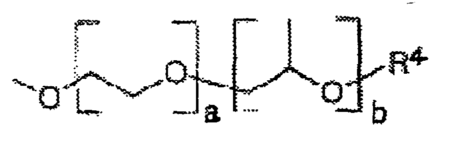
- R³ est choisi parmi CH₃ ou le groupement R²,
- R⁴ est choisi parmi l'hydrogène, CH₃, ou les groupements 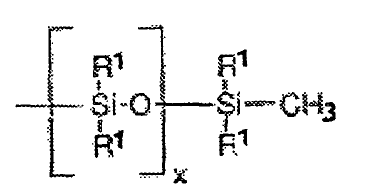 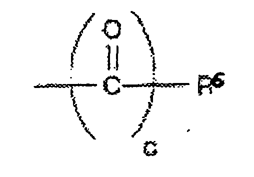
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 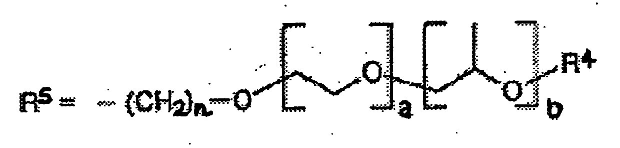
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

De préférence, R¹ est choisi parmi les groupements méthyl, éthyl, propyl, butyl, isobutyl, pentyl, isopentyl, hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux R⁴ ayant pour formule -(CO)c-R⁶, R⁶ étant un alkyl, cycloalkyl or aryl ayant 1 à 40 atomes de carbone qui peut contenir des groupements supplémentaires tels que NH₂, COOH et/ou S0₃H.

On préfère les radicaux R⁶ pour lesquels c=0 qui sont des phosphates ou des sulfates.

Les dérivés siliconés (b) particulièrement préférés sont ceux présentant la formule générale suivante :

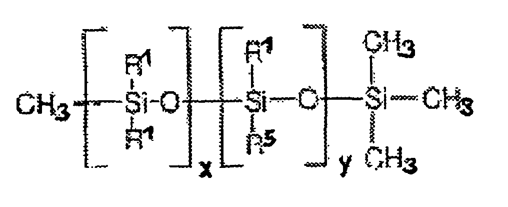

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l.

La composition comprend avantageusement, en pourcentage relatif en poids de la composition, de 0,1 à 20 % de copolymère silicone/acrylate, et plus préférentiellement de 0,5 à 10 % de ce copolymère.

La composition comprend avantageusement, en pourcentage relatif en poids de la composition, de 0,05 à 10 % d'agent épaississant non cellulosique, et de préférence, de 0,1 à 5 %

Au sens de la présente invention, on entend par « épaississant non cellulosique », un épaississant dont la formule chimique est exempte de groupements cellulosiques. Les agents épaississants convenant pour l'invention sont les gommes de xanthane, de scléroglucane, de gellane, de rhamsan, les alginates, la maltodextrine, l'amidon et ses dérivés, la gomme de karaya, la farine de caroube, les gommes de guar.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les tensioactifs anioniques, cationiques, non ioniques ou amphotères autres que ceux de l'invention, les parfums, les filtres, les conservateurs, les protéines, les vitamines, les polymères autres que ceux de l'invention, les huiles végétales, minérales ou synthétiques et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'inventions peuvent être appliquées sur la peau, les ongles, les lèvres, les cheveux, les sourcils et les cils.

Les compositions conformes à l'invention sont particulièrement adaptés pour des cheveux secs ou humides, en tant que produits de coiffage.

## Revendications

1. Composition cosmétique comprenant, dans un milieu cosmétiquement acceptable, au moins un copolymère silicone/acrylate et au moins un agent épaississant non cellulosique, **caractérisée en ce que** le copolymère silicone/acrylate est obtenu par polymérisation radicalaire d'au moins un monomère éthyléniquement insaturé (a) en présence d'au moins un dérivé siliconé (b) comprenant des groupements oxyalkylénés, l'agent épaississant étant choisi parmi les gommes de xanthane, de scléroglucane, de gellane, de rhamsan, les alginates, la maltodextrine, l'amidon et ses dérivés, la gomme de karaya, la farine de caroube, les gommes de guar

2. Composition selon la revendication 1, **caractérisée par le fait que** le monomère (a) répond à la formule (Iₐ) : 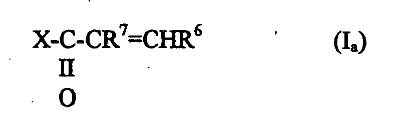 dans laquelle:
- X est choisi dans le groupe comprenant OH, OM, OR⁸, NH₂, NHR⁸, N(R⁸)₂ ;
- M est un cation choisi parmi Na⁺, K⁺, Mg⁺⁺, NH ⁴⁺, alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, le N,N-diméthylaminoéthyl, les groupements 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

3. Composition cosmétique selon la revendication 1, **caractérisée par le fait que** le dérivé siliconé (b) répond à la formule I ci-après : 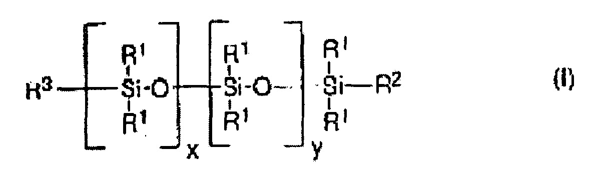 dans laquelle formule (I):
- R² est choisi parmi CH₃ ou le groupement 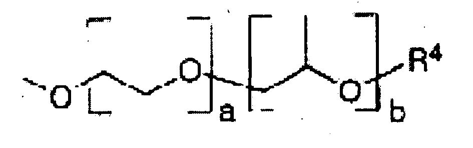
- R³ est choisi parmi CH₃ ou le groupement R²,
- R⁴ est choisi parmi l'hydrogène, CH₃, ou les groupements 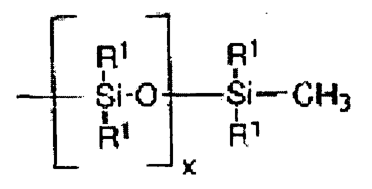 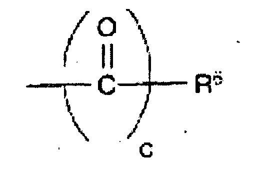
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 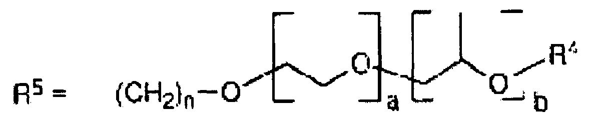
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1

4. Composition selon la revendication 1, **caractérisée par le fait que** le monomère (a) est choisi parmi les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyls ou allyls, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

5. Composition selon la revendication 1, **caractérisée par le fait que** le monomère (a) est choisi dans le groupe comprenant l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Composition selon l'une quelconque des revendications précédentes, **caractérisée par le fait que** les dérivés siliconés (b) sont choisis parmi les diméthicone copolyols ou les tensioactifs siliconés.

7. Composition selon l'une quelconque des revendications précédentes, **caractérisée par le fait qu'**elle comprend, en pourcentage relatif en poids, de 0,1 à 20 % de copolymère silicone/acrylate, et plus préférentiellement de 0,5 à 10 % de ce copolymère.

8. Composition selon l'une quelconque des revendications précédentes, **caractérisée par le fait qu'**elle comprend, en pourcentage relatif en poids, de 0,05 à 10 % d'agent épaississant non cellulosique, et de préférence, de 0,1 à 5 % .

9. Composition selon l'une quelconque des revendications précédentes, **caractérisée par le fait qu'**elle contient en outre au moins un additif choisi parmi les tensioactifs anioniques, cationiques, non ioniques ou amphotères, les parfums, les conservateurs, les protéines, les vitamines, les polymères autres que ceux de l'invention, les huiles minérales ou synthétiques, les filtres et les huiles végétales.

10. Composition selon l'une quelconque des revendication précédentes, **caractérisée par le fait qu'**elle se présente sous la forme d'un gel.

11. Procédé de maintien ou de mise en forme de la coiffure, **caractérisé par le fait qu'**il comprend la mise en oeuvre d'une composition conforme à l'une quelconque des revendications 1 à 10.

12. Utilisation d'une composition selon l'une quelconque des revendications 1 à 10, pour la fabrication d'un produit cosmétique, en particulier capillaire.

13. Utilisation d'une composition selon l'une quelconque des revendications 1 à 10 pour les cheveux, les sourcils et les cils.

## Claims

1. Cosmetic composition comprising, in cosmetically acceptable medium, at least one silicone/acrylate copolymer and at least one non-cellulose-based thickener, **characterized in that** the silicone/acrylate copolymer obtained by radical polymerization of at least one ethylenically unsaturated monomer (a) in the presence of at least one silicone derivative (b) comprising oxyalkylene groups, the thickened being chosen from xanthan gum, scleroglucan gum, gellan gum, rhamsan gum, alginates, maltodextrin, starch and derivatives thereon, gun karaya, carob flour and guar gums.

2. Composition according to Claim 1, **characterised in that** the monomer (a) correspond to formula (Iₐ) : 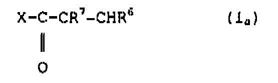 in which:
- X is chosen from the group comprising OH, OM, OR⁸, NH₂, NHR⁸ and N(R⁸)₂;
- M is a nation chosen from Na⁺, K⁺, Mg⁺⁺, NH⁴⁺, alkylammonium, dialkylammonium, trialkylammonium and tetraalkylammonium;
- the radicals R⁸ may be identical or different and are chosen from the group comprising hydrogen, linear or branched C₁ to C₄₀ alkyl groups, mono- or polyhydroxylaled C₁ to C₄₀ alkyl groups, optionally substituted with one or more alkoxy, amino or carboxyl groups, hydroxylated polyethers, N,N-dimethylaminoethyl, 2-hydroxyethyl, 2-methoxyethyl, 2-ethoxyethyl, hydroxypropyl, methoxypropyl and ethoxypropyl groups;
- R⁷ and R⁶ are chosen, independently of each other, from the group comprising hydrogen, linear or branched C₁ to C₈ alkyl groups, methoxy, ethoxy, 2-hydroxyethoxy, 2-methoxyethoxy and 2-ethoxyethyl groups, and CN, COOH and COOM groups.

3. Cosmetic composition according to Claim 1, **characterized in that** the silicone derivative (b) corresponds to the formula (I) below: 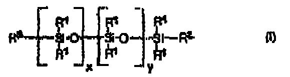 in which formula (T):
- R² is chosen from CH₃ and the group 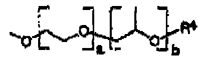
- R³ is chosen from CH₃ and the group R²,
- R⁴ is chosen from hydrogen, CH₃ and the groups 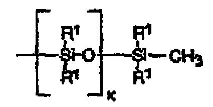 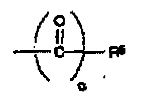
- R⁶ is an organic C₁ to C₄₀ group which can contain amino, carboxyl or sulfonate groups, and if c-0, R₆ is the anion of an inorganic acid;
- the radicals R¹ may be identical or different and are chosen from C₁ to C₂₀ aliphatic hydrocarbons, C₃ to C₂₀ aliphatic or cycloaliphatic hydrocarbons, and the groups R⁵ corresponding to the formula below: 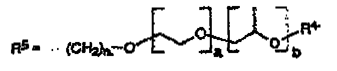
- n is an integer between 1 and 6,
- x and y are integers chosen such that the molecular weight of the polysiloxane is between 300 and 30,000,
- a and b are integers between 0 and 50, and
- c is 0 or 1.

4. Composition according to Claim 1, **characterized in that** the monomer (a) is chosen from C₁ to C₄₀ vinyl and allyl ester, linear C₃ to C₄₀ carboxylic acids or C₃ to C₄₀ carboxycyclic acids, vinyl or allyl haldides, vinyllactams, preferably vinylpyrrolidone and vinylcaprolactam, heterocyclic compound substituted with vinyl or allyl groups, preferably vinylpyridine, vinyloxazoline and allylpyridine, N-vinylimidazoles, diallylamines, vinylidene chlorine, carbon-based unsaturated compounds, such as styrene or isoprene, and acrylic or methacrylic acid derivatives quaternized with epichlorohydrin.

5. Composition according to Claim 1, **characterized in that** the monomer (a) is chosen from the group comprising acrylic, methacrylic and ethacrylic acid; methyl, ethyl, propyl, n-butyl, isobutyl, t-butyl, 2-ethylhexyl and decyl acrylate; methyl, ethyl, propyl, n-butyl, isobutyl, t-butyl, 2-ethylhexyl and decyl methacrylale; methyl, ethyl, propyl, n-butyl, isobutyl, t-butyl, 2-ethylhexyl and docyl ethacrylate; 2,3-dihydroxypropyl acrylate; 2,3-dihydroxypropyl methacrylate; 2-dihydroxyethyl acrylate; hydroxypropyl acrylate; 2-hydroxyethyl methacrylate; 2-hydroxyethyl ethacrylate; 2-methoxyethyl acrylate; 2-ethoxyethyl methacrylate; 2-ethoxyethyl ethacrylate; hydroxypropyl methacrylate, glyceryl monoacrylate; glyceryl monomethacrylate, polyalkylene glycol (meth)acrylates, unsaturated sulfonic acids, acrylamide, methacrylamide, ethacrylamide, N,N-dimethylacrylamide, N-ethylacrylamide, N-ethylmethacrylamide, 1-vinylimidazole, N,N-dimethylaminoethyl (meth)acrylate, maleic acid, fumaric acid, maleic anhydride and its monoesters, crotonic acid, itaconic acid, vinyl ethers, vinylformamide, vinylamine, vinylpyridine, vinylimidazole, vinylfuran, styrene, styryl sulfonate and allyl alcohol, and mixtures thereof.

6. Composition according to any one of the preceding claims, **characterized in that** the silicone derivatives (b) are chosen from dimethicone copolyols and silicone surfactants.

7. Composition according to any one of the preceding claims, **characterised in that** it comprises, as a relative percentage by weight, from 0.1% to 20% of silicone/acrylate copolymer, and more preferable from 0.5% to 10% of this copolymer.

8. Composition according to any one of the preceding claims, **characterized in that** it comprises, as a relative percentage by weight, from 0.05% to 10% of non-cellulose-based thickener and preferably from 0.1% to 5%.

9. Composition according to any one of the preceding claims, **characterized in that** it also contains at least one additive chosen from anionic, cationic, nonionic or amphoteric surfactants, fragrances, preserving agents, proteins, vitamins, polymers other than those of the invention, mineral or synthetic oils, screening agents and plant oils.

10. Composition according to any one of the preceding claims, **characterized in that** it is in the form of a gel.

11. Process for holding or shaping the hairstyle, **characterized in that** it comprises the use of a composition in accordance with any one of Claims 1 to 10.

12. Use of composition according to any one of Claims 1 to 10, for the manufacture of a cosmetic product, in particular a hair product.

13. Use of a composition according to any one of Claims 1 to 10, for the hair, the eyebrows and the eyelashes.

## Patentansprüche

1. Kosmetische Zusammensetzung, die in einem kosmetisch akzeptablen Medium mindestens ein Silicon/Acrylat-Copolymer und mindestens ein Verdickungsmittel enthält, das nicht auf Cellulose basiert, **dadurch gekennzeichnet, dass** das Silicon/Acrylat-Copolymer durch radikalische Polymerisation mindestens eines ethylenisch ungesättigten Monomers (a) in Gegenwart mindestens eines Siliconderivats (b), das alkoxylierte Gruppen aufweist, erhalten wird, wobei das Verdickungsmittel unter Xanthan, Skleroglucan, Gellan, Rhamsan, Alginaten, Maltrodextrin, Stärke und ihren Derivaten, Karaya-Gummi, Johannisbrotkernmehl und Guargummen ausgewählt ist.

2. Zusammensetzung nach Anspruch 1, **dadurch gekennzeichnet, dass** das Monomer (a) der Formel (Iₐ) entspricht: 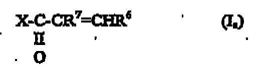 in der Formel:
- X ist ausgewählt unter OH, OM, OR⁸, NH₂, NHR⁸, N(R⁸)₂;
- M ist ein Kation, das unter Na⁺, K⁺, Mg⁺⁺, NH⁴⁺, Alkylammonium, Dialkylammonium, Trialkylammonium und Tetraalkylammonium ausgewählt ist;
- die Gruppen R⁸ können gleich oder verschieden sein und sind ausgewählt unter Wasserstoff, geradkettigen oder verzweigten Alkylgruppen mit 1 bis 40 Kohlenstoffatomen, ein- oder mehrfach hydroxylierten Alkylgruppen mit 1 bis 40 Kohlenstoffatomen, die gegebenenfalls mit einer oder mehreren Alkoxy-, Amino- oder Carboxygruppe(n) substituiert sind, hydroxylierten Polyethergruppen, N,N-Dimethylaminoethyl, 2-Hydroxyethyl, 2-Methoxyethyl, 2-Ethoxyethyl, Hydroxypropyl, Methoxypropyl und Ethoxypropyl;
- R⁷ und R⁶ sind unabhängig voneinander ausgewählt unter Wasserstoff, linearen oder verzweigten Alkylgruppen mit 1 bis 8 Kohlenstoffatomen, Methoxy, Ethoxy, 2-Hydroxyethoxy, 2-Methoxyethoxy und 2-Ethoxyethyl, CN, COOH und COOM.

3. Kosmetische Zusammensetzung nach Anspruch 1, **dadurch gekennzeichnet, dass** das Siliconderivat (b) der folgenden Formel (I) entspricht: 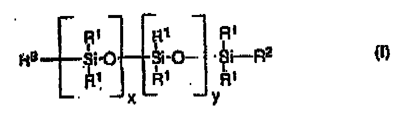 wobei in der Formel (I):
- R² ist unter CH₃ oder der folgenden Gruppe ausgewählt: 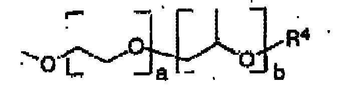
- R³ ist unter CH₃ oder der Gruppe R² ausgewählt,
- R⁴ ist unter Wasserstoff, CH₃ oder den folgenden Gruppen ausgewählt: 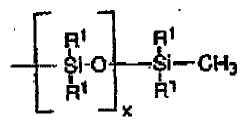 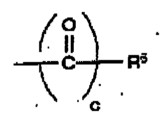
- R⁶ ist eine organische Gruppe mit 1 bis 40 Kohlenstoffatomen, die Amino-, Carboxy- oder Sulfonatgruppen enthalten kann, und, wenn c=0, ist R₆ das Union einer anorganischen Säure;
- die Gruppen R¹ können gleich oder verschieden sein und sind unter den aliphatischen Kohlenwasserstoffen mit 1 bis 20 Kohlenstoffatomen, aliphatischen oder cycloaliphatischen Kohlenwasserstoffen mit 3 bis 20 Kohlenstoffatomen und den Gruppen R⁵ ausgewählt, die der folgenden Formel entsprechen: 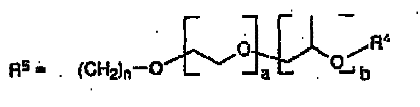
- n ist eine ganze Zahl im Bereich von 1 bis 6,
- x und y sind ganze Zahlen, die so ausgewählt sind, dass das Molekulargewicht des Polysiloxans im Bereich von 300 bis 30 000 liegt,
- a und b sind ganze Zahlen im Bereich von 0 bis 50, und
- c ist 0 oder 1.

4. Zusammensetzung nach Anspruch 1, **dadurch gekennzeichnet, dass** das Monomer (a) unter Vinylestern und Allylestern mit 1 bis 40 Kohlenstoffatomen, linearen Carbonsäuren mit 3 bis 40 Kohlenstoffatomen oder carboxycyclischen Carbonsäuren mit 3 bis 40 Kohlenstoffatomen, Vinylhaliden oder Allylhaliden, Vinyllactamen und vorzugsweise Vinylpyrrolidon und Vinylcaprolactam, mit Vinyl- oder Allylgruppen substituierten heterocyclischen Verbindungen, vorzugsweise Vinylpyridin, Vinyloxazolin und Allylpyridin, N-Vinylimidazolen, Diallylaminen, Vinylidenchlorid, ungesättigten Verbindungen auf Kohlenstoffbasis, wie Styrol oder Isopren, mit Epichlorhydrin quaternisierten Derivaten von Acrylsäure oder Methacrylsäure ausgewählt ist.

5. Zusammensetzung nach Anspruch 1, **dadurch gekennzeichnet, dass** das Monomer (a) ausgewählt ist unter Acrylsäure, Methacrylsäure und Ethacrylsäure; Methylacrylat, Ethylacrylat, Propylacrylat, *n*-Butylacrylat, Isobutylacrylat, *t*-Butylacrylat, 2-Ethylhexylacrylat und Decylacrylat; Methymethacrylat, Ethylmethacrylat, Propylmethacrylat, *n*-Butylmethacrylat, Isobutylmethacrylat, *t*-Butylmethacrylat, 2-Ethylhexylmethacrylat und Decylmethacrylat; Methylethacrylat, Ethylethacrylat, Propylethacrylat, *n*-Butylethacrylat, Isobutylethacrylat, *t*-Butylethacrylat, 2-Ethylhexylethacrylat und Decylethacrylat; 2,3-Dihydroxypropylacrylat; 2,3-Dihydroxypropylmethacrylat; 2-Dihydroxyethylacrylat; Hydroxypropylacrylat; 2-Hydroxyethylmethacrylat; 2-Hydroxyethylethacrylat; 2-Methoxyethylacrylat; 2-Ethoxyethylmethacrylat; 2-Ethoxyethylethacrylat; Hydroxypropylmethacrylat; Glycerylmonoacrylat; Glycerylmonomethacrylat Polyalkylenglycol(meth)acrylaten, ungesättigten Sulfonsäuren, Acrylamid, Methacrylamid, Ethacrylamid, N,N-Dimethylacrylamid, N-Ethylacrylamid, N-Ethylmethacrylamid, 1-Vinylimidazol, N,N-Dimethylaminoethyl(meth)acrylat, Maleinsäure, Fumarsäure, Maleinsäureanhydrid und seinen Monoestern, Crotonsäure, Itaconsäure, Vinylethern, Vinylformamid, Vinylamin, Vinylpyridin, Vinylimidazol, Vinylfuran, Styrol, Styrolsulfonat, Allylalkohol und deren Gemischen.

6. Zusammensetzung nach einem der vorhergehenden Ansprüche, **dadurch gekennzeichnet, dass** die Siliconderivate (b) unter den Dimethiconcopolyolen oder siliconierten grenzflächenaktiven Stoffen ausgewählt sind.

7. Zusammensetzung nach einem der vorhergebenden Ansprüche, **dadurch gekennzeichnet, dass** sie, ausgedrückt als relativer Gewichtsanteil, 0,1 bis 20 % Silicon/Acrylat-Copolymer und noch bevorzugter 0,5 bis 10% dieses Copolymers enthält.

8. Zusammensetzung nach einem der vorhergehenden Ansprüche, **dadurch gekennzeichnet, dass** sie, ausgedrückt als relativer Gewichtsanteil, 0,05 bis 10 % Verdickungsmittel, das nicht auf Cellulose basiert, und vorzugsweise 0,1 bis 5 % Verdickungsmittel enthält.

9. Zusammensetzung nach einem der vorhergehenden Ansprüche, **dadurch gekennzeichnet, dass** sie ferner mindestens einen Zusatzstoff enthält, der unter den anionischen, kationischen, nichtionischen amphoteren grenzflächenaktiven Stoffen, Parfums, Konservierungsmitteln, Proteinen, Vitaminen, Polymeren, die von den erfindungsgemäßen Polymeren verschieden sind, Mineralölen oder synthetischen Ölen, Filtern und pflanzlichen Ölen ausgewählt ist.

10. Zusammensetzung nach einem der vorhergehenden Ansprüche, **dadurch gekennzeichnet, dass** sie in Form eines Gels vorliegt.

11. Verfahren für die Festigung oder Formgebung der Frisur, **dadurch gekennzeichnet, dass** es die Verwendung einer Zusammensetzung nach einem der Ansprüche 1 bis 10 umfasst.

12. Verwendung einer Zusammensetzung nach einem der Ansprüche 1 bis 10 für die Herstellung eines kosmetischen und insbesondere haarkosmetischen Produkts.

13. Verwendung einer Zusammensetzung nach einem der Ansprüche 1 bis 10 für die Haare, die Augenbrauen und die Wimpern.
